# EUROPÄISCHE PATENTSCHRIFT

(11) **EP 1 484 072 B1**
(45) Veröffentlichungstag und Bekanntmachung des Hinweises auf die Patenterteilung: **19.09.2007**
(21) Anmeldenummer: 04011086.8
(22) Anmeldetag: 10.05.2004
(51) Int. Cl.: A61M 5/315

(54) **Spritze sowie Verfahren zum Herstellen einer solchen**
Syringe and a method for manufacturing the same
Seringue et procédé de fabrication d'une telle seringue

(30) Priorität: 04.06.2003 DE 10326706
(43) Veröffentlichungstag der Anmeldung: 08.12.2004
(73) Patentinhaber: Schott AG, 55122 Mainz (DE)
(72) Erfinder: Wolbring, Peter, Dr., 66606 St. Wendel (CH); Mosimann, Ralph, 9514 Wuppenau (CH); Diem, Hanspeter, 9100 Herisau (CH)
(74) Vertreter: Duhme, Torsten

(56) Entgegenhaltungen:
- EP-A- 0 764 450
- DE-A- 3 107 414
- PATENT ABSTRACTS OF JAPAN Bd. 2003, Nr. 11, 5. November 2003 (2003-11-05) -& JP 2003 199827 A (TERUMO CORP), 15. Juli 2003 (2003-07-15)

## Beschreibung

Die vorliegende Erfindung betrifft ein Verfahren zum Herstellen einer Spritze, insbesondere für medizinische Anwendungen, mit den Schritten:
a) Bereitstellen eines Spritzenzylinders mit einem proximalen und einem distalen Ende,
b) Einsetzen eines Kolbenstopfens in den Spritzenzylinder, und
c) Anordnen eines Rückanschlags am proximalen Ende des Spritzenzylinders, wobei der Rückanschlag mit einer Kolbenstange zu einem kombinierten Montageteil verbunden wird und im Schritt c) von der Kolbenstange abgetrennt und am Spritzenzylinder befestigt wird.

Die Erfindung betrifft ferner eine Spritze, insbesondere für medizinische Anwendungen, mit einem Spritzenzylinder, einem Kolbenstopfen, einer daran befestigten Kolbenstange und mit einem Rückanschlag für den Kolbenstopfen, wobei der Rückanschlag an einem proximalen Ende des Spritzenzylinders angeordnet ist, und wobei der Rückanschlag und die Kolbenstange als kombiniertes Montageteil ausgebildet sind, das getrennt an dem Spritzenzylinder angeordnet ist.

Ein solches Verfahren und eine entsprechende Spritze sind aus DE 31 07 414 A1 bekannt.

Im Bereich der pharmazeutischen Industrie gibt es eine Reihe von Medikamenten, die bereits herstellerseitig in medizinische Spritzen abgefüllt werden. Der pharmazeutische Hersteller füllt das Medikament unter sterilen Bedingungen in einen sterilen, distal mit einer dichtenden Kappe verschlossenen Spritzenzylinder. Anschließend wird der Spritzenzylinder am proximalen Ende mit einem sterilen Kolbenstopfen, häufig aus gummielastischem Material, verschlossen. Daran anschließend wird üblicherweise die Kolbenstange der Spritze in ein entsprechend vorgesehenes Innengewinde am Kolbenstopfen eingeschraubt, die Spritze steril verpackt und endkonfektioniert. Ein besonderer Vorteil dieser Vorgehensweise ist, dass das Medikament auf diese Weise bis zur eigentlichen Applikation steril gehalten ist, was beispielsweise dann, wenn das Medikament erst unmittelbar vor der Applikation in die Spritze aufgezogen wird, nicht immer gewährleistet ist.

Aus EP 0 764 450 B1 ist eine Spritze bekannt, die sich für derartige Anwendungszwecke gut eignet, da der vorhandene Rückanschlag am proximalen Ende des Spritzenzylinders verhindert, dass der Kolbenstopfen nach dem Befüllen und Zusammenbau der Spritze nach hinten, d.h. in Richtung des proximalen Endes, herausgezogen werden kann. Ein versehentliches Entfernen des Kolbenstopfens, was die Sterilität gefährden würde, ist auf diese Weise ausgeschlossen. Darüber hinaus eignet sich eine solche Spritze mit Rückanschlag besonders für sehr teure und/oder hochgiftige Medikamente, beispielsweise Krebsmittel, da bei einem versehentlichen Hinausziehen des Kolbenstopfens das in der Spritze enthaltene Medikament austreten könnte und so eine Gefährdung für das medizinische Personal darstellen kann oder zumindest einen teuren Verlust bedeutet.

Ein weiterer bevorzugter Anwendungsfall für Spritzen mit Rückanschlag findet sich, wenn das zu applizierende Medikament durch mehrfaches Aufziehen auf die Spritze aufbereitet werden muss. Auch in diesen Fällen wird durch den Rückanschlag verhindert, dass der Kolbenstopfen versehentlich zu weit nach hinten und damit aus dem Spritzenzylinder hinausgezogen werden kann.

Bei der bekannten Spritze besteht der Rückanschlag aus einer Griffplatte und einem daran angeformten Flansch. Der Rückanschlag wird nach Einsetzen des Kolbenstopfens in den Spritzenzylinder seitlich, d.h. orthogonal zur Längsrichtung der Spritze und damit orthogonal zur Bewegungsrichtung des Kolbenstopfens, auf den Spritzenzylinder aufgesteckt. Die Fingerplatte und der Flansch weisen eine seitliche Ausnehmung auf, die dieses seitliche Aufstecken oder Aufschnappen auf den Außenumfang des Spritzenzylinders ermöglichen. Des Weiteren besitzen die Fingerplatte und der Flansch am äußeren proximalen Ende des Spritzenzylinders einen Innendurchmesser, der geringer ist als der Außendurchmesser des Kolbenstopfens, wodurch der Rückanschlag gebildet wird.

Aus der WO 99/55402 ist eine Spritze bekannt, bei der der Rückanschlag durch einen im Inneren des Spritzenzylinders angeordneten Vorsprung gebildet wird. An der Kolbenstange der Spritze sind speziell ausgeformte Rasthaken vorgesehen, die in Vorschubrichtung der Kolbenstange den Vorsprung überschnappen können, während sie in Gegenrichtung blockieren.

Aus der WO 94/26334 ist eine Spritze bekannt, bei der als Rückanschlag ein Flansch verwendet wird, der am proximalen Ende des Spritzenzylinders von der Seite her aufgesteckt wird. Der Flansch besitzt eine in das Innere des Spritzenzylinders hineinragende Lasche, die ein Herausziehen des Kolbenstopfens verhindert.

Darüber hinaus gibt es eine Vielzahl von weiteren Alternativen, um bei einer Spritze ein unbeabsichtigtes Herausziehen des Kolbenstopfens nach hinten mit Hilfe eines Rückanschlags zu verhindern. Beispielhaft sei auf die WO 94/13339, die WO 00/07648, die JP 2001-327600 A, die US 5,250,030, die DE 44 34 644 C2 oder die EP 0 738 517 B1 verwiesen. Ein großer Teil der bekannten Lösungen erfordert allerdings im Vergleich zu Spritzen ohne Rückanschlag Veränderungen und/oder Ergänzungen am Spritzenzylinder und/oder an der Kolbenstange. Dies verhindert, dass "konventionelle" Spritzen mit einem entsprechenden Rückanschlag ausgerüstet werden können. Als Folge davon sind derartige "Spezialanfertigungen" erheblich teurer als herkömmliche Spritzen ohne Rückanschlag.

Der aus der oben genannten EP 0 764 450 B1 bekannte Rückanschlag kann demgegenüber zwar bei standardmäßig ausgebildeten Spritzenzylindern, insbesondere bei solchen mit Rundflansch, verwendet werden. Die Montage des Rückanschlags erfolgt hierbei jedoch in einem separaten Montageschritt und zudem in einer Bewegungsrichtung, die quer zu der Bewegungsrichtung verläuft, in der der Kolbenstopfen in den Spritzenzylinder eingesetzt wird. Durch den zusätzlichen und vom übrigen Bewegungsablauf abweichenden Montageschritt entstehen ebenfalls Zusatzkosten, die über die reinen Materialkosten für den Rückanschlag hinausgehen.

Die eingangs genannte DE 31 07 414 A1 offenbart eine Einmalspritze für den medizinischen Gebrauch. Die Spritze besitzt einen Spritzenzylinder, der am proximalen Ende mit einer Scheibe verschlossen wird, um die Gefahr von Kontaminationen an den Innenwänden des Spritzenzylinders zu verringern. Die Scheibe ist einstückig an einer Kolbenstange angeformt, welche vom proximalen Ende des Spritzenzylinders her in diesen eingeschoben wird. Am distalen Ende der Kolbenstange ist in herkömmlicher Weise ein Kolbenstopfen angeformt, der zusammen mit der Kolbenstange und der Scheibe ein einstückiges Montageteil bildet. Beim Einschieben dieses Montageteils in den Spritzenzylinder rastet die Scheibe in eine Rasteinrichtung am proximalen Ende des Spritzenzylinders ein und reißt dabei von der Kolbenstange ab. Die bekannte Einmalspritze ist ein einfacher Wegwerfartikel, der aus lediglich zwei Form- oder Montageteilen zusammengesetzt wird und dementsprechend kostengünstig ist.

Vor diesem Hintergrund ist es eine Aufgabe der vorliegenden Erfindung, ein alternatives Verfahren anzugeben, mit dem sich Spritzen mit Rückanschlag für eine herstellerseitige Befüllung kostengünstig fertigen lassen.

Diese Aufgabe wird nach einem Aspekt der Erfindung mit einem Verfahren der eingangs genannten Art gelöst, bei dem die Kolbenstange im Schritt c) mit einer Drehbewegung in den Kolbenstopfen eingeschraubt wird und bei dem der Rückanschlag mit der Drehbewegung von der Kolbenstange getrennt wird.

Die Aufgabe wird nach einem weiteren Aspekt der Erfindung durch eine Spritze der eingangs genannten Art gelöst, bei der die Kolbenstange mit einer Drehbewegung in den Kolbenstopfen eingeschraubt ist und bei der das kombinierte Montageteil so ausgebildet ist, dass der Rückanschlag mit der Drehbewegung von der Kolbenstange abgetrennt ist.

Gemäß einem weiteren Aspekt der Erfindung wird diese Aufgabe durch Bereitstellen eines Montageteils zum Herstellen einer Spritze gelöst, wobei das Montageteil einen Rückanschlag und eine Kolbenstange mit einem Gewindezapfen aufweist, der zum Einschrauben in einen Kolbenstopfen ausgebildet ist, und wobei eine vorbereitete Trennstelle so ausgebildet ist, dass der Rückanschlag mit einer Drehbewegung von der Kolbenstange abgetrennt werden kann.

Nach dem neuen Verfahren bilden der Rückanschlag und die Kolbenstange somit vor der abschließenden Montage der Kolbenstange eine Einheit, die erst im Verlauf der Montage getrennt wird. Auf diese Weise ist es möglich, den Rückanschlag ohne zusätzlichen Transportvorgang an den Spritzenzylinder heranzuführen, was die Anzahl der Montageschritte reduziert. Darüber hinaus ist für das Heranführen des Rückanschlags an den Spritzenzylinder kein gesondertes Maschinenteil erforderlich, was ebenfalls zu einer Kostenreduzierung beiträgt, da die neuen Spritzen im Wesentlichen auf derselben Maschine montiert werden können, wie herkömmliche Spritzen ohne Rückanschlag. Das neue Verfahren lässt sich einfach und kostensparend in bestehende Produktionsabläufe integrieren. Ein weiterer Vorteil ist, dass der Rückanschlag und die Kolbenstange aufgrund des neuen Verfahrens in einem gemeinsamen Herstellungsprozess gefertigt werden können, was die Anzahl der erforderlichen Herstellungsschritte noch weiter reduziert.

Das neue Verfahren und die weiteren Ausprägungen der Erfindung beruhen unter anderem auf der Idee, den Rückanschlag und die Kolbenstange zunächst zu kombinieren und erst im Verlauf der Montage voneinander abzutrennen. In besonders bevorzugten Ausgestaltungen, die nachfolgend näher erläutert werden, ist sogar vorgesehen, dass der Rückanschlag und die Kolbenstange materialschlüssig, d.h. einstückig, zu dem kombinierten Montageteil verbunden sind. Dies bedingt, dass das Abtrennen ein Abreißen oder Aufbrechen der materialschlüssigen Verbindung beinhaltet. Ein solcher Schritt ist im Bereich der pharmazeutischen Industrie und insbesondere bei dem eingangs beschriebenen Befüllen von Spritzen unter sterilen Bedingungen ungewöhnlich und unüblich, da bei jedem Aufbrechen/Aufreißen einer Verbindung mikrokleine Partikel freigesetzt werden, die zu einer Verunreinigung des Medikaments (oder auch eines anderen Stoffes, der unter hohen Reinheitsbedingungen hergestellt und verpackt werden soll) führen kann. Im vorliegenden Fall wird jedoch die neue Erkenntnis ausgenutzt, dass eventuell entstehende Partikel das steril abgefüllte Medikament nicht verunreinigen können, da zu dem Zeitpunkt, zu dem die Trennung von Kolbenstange und Rückanschlag erfolgt, der Spritzenzylinder bereits durch den Kolbenstopfen verschlossen ist. Entgegen aller üblichen Gepflogenheiten ist es in diesem speziellen Fall daher möglich, sogar eine materialschlüssige Verbindung aufzutrennen, ohne die für den pharmazeutischen Bereich kritischen Reinheitsbedingungen zu gefährden. Grundsätzlich ist anstelle einer materialschlüssigen Verbindung jedoch auch eine form- oder kraftschlüssige Verbindung von Rückanschlag und Kolbenstange möglich, ohne auf die oben beschriebene Reduzierung der Montageschritte verzichten zu müssen.

Die genannte Aufgabe ist daher vollständig gelöst.

In einer bevorzugten Ausgestaltung der Erfindung wird der Rückanschlag im Schritt c) im Wesentlichen parallel zu einer Längsachse des Spritzenzylinders an diesen herangeführt. Der Rückanschlag ist dabei vorzugsweise konzentrisch mit der Kolbenstange zu dem kombinierten Montageteil verbunden.

Durch diese Ausgestaltung kann die Anzahl der erforderlichen Montageschritte bzw. die Anzahl der erforderlichen Bewegungsabläufe noch weiter reduziert werden. Genau genommen können der Rückanschlag und die Kolbenstange in dieser Ausgestaltung in einer einzigen geradlinigen Bewegung an den Spritzenzylinder herangeführt werden, was exakt dem Bewegungsablauf entspricht, wie er bei der Herstellung von herkömmlichen Spritzen üblich ist. Das neue Verfahren lässt sich daher in dieser Ausgestaltung noch einfacher in herkömmliche Produktionsabläufe integrieren, was den Aufwand und die Kosten noch weiter reduziert.

In einer weiteren Ausgestaltung ist der Rückanschlag mit der Kolbenstange über Sollbruchstellen derartig verbunden, dass der Rückanschlag beim Befestigen der Kolbenstange am Kolbenstopfen von der Kolbenstange abreißt.

Entsprechend dieser bevorzugten Ausgestaltung des neuen Verfahrens weisen der Rückanschlag und die Kolbenstange vorzugsweise jeweils Abrissstellen auf, die zusammen zumindest eine Sollbruchstelle festlegen.

Nach dieser bevorzugten Ausgestaltung sind der Rückanschlag und die Kolbenstange einstückig miteinander verbunden. Dies ermöglicht es, Rückanschlag und Kolbenstange in einem einzigen Spritzgießvorgang herzustellen, was zu einer besonders deutlichen Kostenreduktion führt. Es ist jedoch auch möglich, Rückanschlag und Kolbenstange in einem zumindest zweistufigen Spritzgießvorgang herzustellen, was den Vorteil bietet, dass der Rückanschlag und die Kolbenstange aus unterschiedlichen Materialien hergestellt werden können. Wie bereits weiter oben erwähnt, ist das Abreißen des Rückanschlags von der Kolbenstange im vorliegenden Fall trotz des sensiblen Einsatzbereichs nicht problematisch, da etwaige Mikropartikel außerhalb des sterilen Bereichs entstehen.

In einer weiteren Ausgestaltung der Erfindung wird der Rückanschlag mit einer bajonettartigen Verriegelung an dem Spritzenzylinder befestigt, wobei die bajonettartige Verriegelung vorzugsweise zumindest eine Halterung aufweist, in die ein vorspringendes Teil eines an dem Spritzenzylinder angeordneten Flansches eingreift.

Diese Ausgestaltung der Erfindung ist besonders vorteilhaft für Spritzen, bei denen der Spritzenzylinder am proximalen Ende einen sogenannten DIN-Flansch aufweist. Der DIN-Flansch, der bei herkömmlichen Spritzen verbreitet ist, ergibt sich gewissermaßen aus einem Rundflansch, von dem zwei gegenüberliegende Kreissegmente an Sekanten abgetrennt sind. Der DIN-Flansch ist somit nicht rotationssymmetrisch, was eine Befestigung mit Hilfe einer bajonettartigen Verriegelung besonders einfach und effizient macht. In einer bevorzugten Ausgestaltung, die nachfolgend anhand eines Ausführungsbeispiels näher beschrieben ist, sind zwei Halterungen am Rückanschlag angeordnet, von denen jeweils eine eine der "Ecken" des DIN-Flansches im Verlauf der Drehbewegung aufnimmt. Ein Abreißen bzw. Abtrennen des Rückanschlags von der Kolbenstange lässt sich in dieser Ausgestaltung besonders einfach realisieren, da die bajonettartige Verriegelung den Drehwinkel des Rückanschlags begrenzt. Durch das "normale" Einschrauben der Kolbenstange in den Kolbenstopfen, das einen deutlich größeren Drehwinkel voraussetzt, ergibt sich dementsprechend zwangsläufig eine Kraft, die zum Abtrennen des Rückanschlags vorteilhaft ausgenutzt werden kann.

In einer weiteren Ausgestaltung führt die Kolbenstange beim Befestigen an dem Kolbenstopfen eine Translationsbewegung aus, wobei der Rückanschlag mit der Translationsbewegung von der Kolbenstange getrennt wird.

Eine Translationsbewegung ist bei herkömmlichen Montagevorgängen ebenfalls standardmäßig vorhanden, da sich die Kolbenstange beim Einschrauben in den Kolbenstopfen auch in Längsrichtung der Spritze bewegt. Auch diese Bewegung kann besonders effizient und damit kostengünstig zum Abtrennen des Rückanschlags ausgenutzt werden, gegebenenfalls auch in Kombination mit der weiter oben bereits erläuterten Drehbewegung. In geeigneten Fällen kann die Translationsbewegung der Kolbenstange jedoch auch für sich genommen zum Abtrennen des Rückanschlags ausgenutzt werden, beispielsweise wenn die Kolbenstange abweichend vom oben beschriebenen Verlauf nicht in den Kolbenstopfen geschraubt wird, sondern anderweitig mit dem Kolbenstopfen verbunden wird.

In einer weiteren Ausgestaltung wird der Rückanschlag mit Schnappelementen an dem Spritzenzylinder befestigt, wobei die Schnappelemente einen am Spritzenzylinder angeordneten Flansch umgreifen. Bevorzugt handelt es sich bei den Schnappelementen um einen segmentierten Ring, der einen am proximalen Ende des Spritzenzylinders angeordneten Rundflansch umgreift und bei Vorschub der Kolbenstange (Translationsbewegung) abreißt.

Diese Ausgestaltung ist besonders vorteilhaft, wenn der Spritzenzylinder an seinem proximalen Ende rotationssymmetrisch ausgebildet ist, da in diesem Fall eine bajonettartige Verriegelung einen zusätzlichen Aufwand darstellen würde. Daher ist diese Ausgestaltung besonders kostengünstig und effizient bei Spritzenzylindern mit Rundflansch.

In einer weiteren Ausgestaltung weist der Rückanschlag zumindest einen flügelartig abstehenden Seitenbereich auf, der eine ergonomische Fingerauflage bildet. In einem derzeit bevorzugten Ausführungsbeispiel besitzt der Rückanschlag beispielsweise einen ellipsenförmigen Kragen, der zwei derartige Flügel ausbildet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine Kolbenstange und einen Rückanschlag als kombiniertes Montageteil gemäß einem ersten Ausführungsbeispiel,
- Fig. 2: eine Spritze unter Verwendung des Montageteils aus Fig. 1,
- Fig. 3: die Spritze aus Fig. 2 in einer Seitenansicht,
- Fig. 4: die Spritze aus Fig. 3 in einer Querschnittsansicht, wobei die Kolbenstange hier bereits weiter in den Spritzenzylinder eingeschoben ist,
- Fig. 5: das Montageteil aus Fig. 1 in einer vergrößerten Querschnittsdarstellung entlang der Schnittlinie V-V in Fig. 1,
- Fig. 6: ein Montageteil bestehend aus Rückanschlag und Kolbenstange gemäß einem weiteren Ausführungsbeispiel,
- Fig. 7: eine Spritze mit dem Montageteil aus Fig. 6,
- Fig. 8: die Spritze aus Fig. 7 in einer Seitenansicht, und
- Fig. 9: die Spritze aus Fig. 8 in einer Querschnittsdarstellung, wobei die Kolbenstange bereits in den Spritzenzylinder eingeschoben ist.

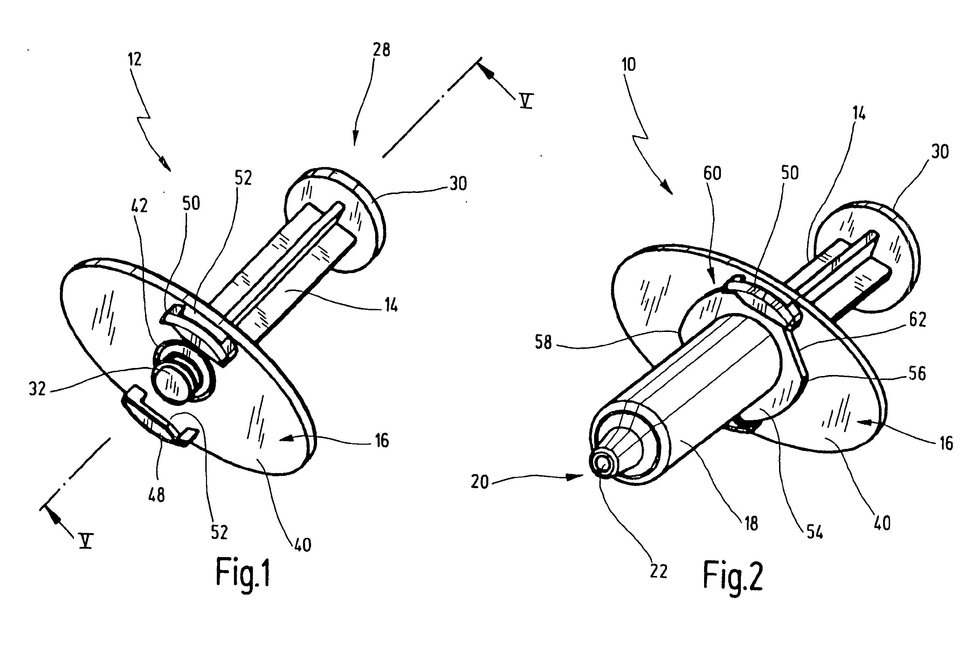

In den Figuren 1 bis 4 ist eine Spritze, die nach dem neuen Verfahren hergestellt ist, in ihrer Gesamtheit mit der Bezugsziffer 10 bezeichnet.

Die Spritze 10 ist unter Verwendung eines Montageteils 12 hergestellt, das die spätere Kolbenstange 14 sowie den späteren Rückanschlag 16 beinhaltet. Kolbenstange 14 und Rückanschlag 16 sind vor der Endmontage der Spritze 12 zu dem in Fig. 1 gezeigten Montageteil materialschlüssig verbunden.

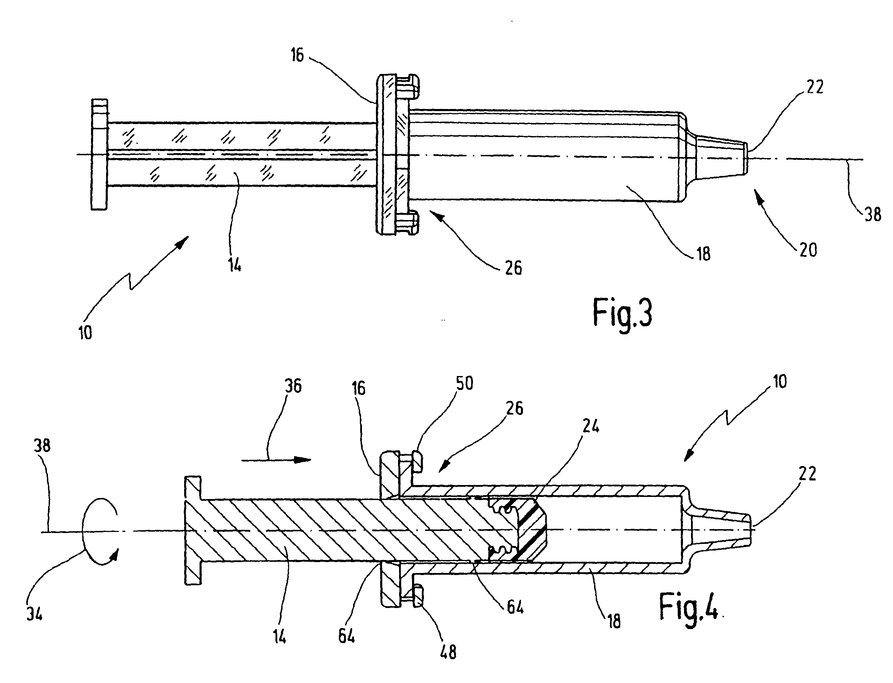

Mit der Bezugsziffer 18 ist ein Spritzenzylinder bezeichnet, der an seinem distalen Ende 20 in an sich bekannter Weise eine Öffnung 22 besitzt. An der Öffnung 22 kann eine Kanüle befestigt werden. Bevorzugt wird die Spritze 10 vor ihrer Endmontage mit einem Medikament (hier nicht dargestellt) befüllt und anschließend mit einem Kolbenstopfen 24 (Fig. 4) am proximalen Ende 26 verschlossen. Dies geschieht bei der Abfüllung von Medikamenten üblicherweise unter sterilen Bedingungen beim pharmazeutischen Hersteller des Medikaments. Der Vollständigkeit halber sei allerdings erwähnt, dass die vorliegende Erfindung nicht allein auf Spritzen für diese Anwendungszwecke beschränkt ist, sondern auch bei allen anderen medizinischen Spritzen und darüber hinaus auch bei Spritzen für den nichtmedizinischen Bereich angewendet werden kann.

Die Kolbenstange 14 besitzt an ihrem proximalen Ende 28 eine Abschlussplatte 30, die bei der Anwendung der Spritze als Auflagefläche beispielsweise für den Daumen des Mediziners dient. An die Auflageplatte 30 schließt sich die eigentliche Kolbenstange an, die an ihrem vorderen Ende in einen Gewindezapfen 32 ausläuft. Im Bereich zwischen Abschlussplatte 30 und Gewindezapfen 32 besitzt die Kolbenstange 14 hier ein im Querschnitt kreuzförmiges Profil, wie dies von gattungsgemäßen Spritzen bekannt ist. Die Erfindung ist hierauf jedoch nicht beschränkt, d.h. die Kolbenstange kann auch ein beliebiges anderes Profil aufweisen.

Wie in Fig. 4 dargestellt, wird die Kolbenstange 14 bei der Endmontage der Spritze 10 in den zuvor in den Spritzenzylinder 18 eingesetzten Kolbenstopfen 24 eingeschraubt. Die hierzu erforderliche Drehbewegung ist in Fig. 4 mit einem Pfeil 34 gekennzeichnet. Außerdem führt die Kolbenstange 14 beim Befestigen am Kolbenstopfen 24 auch eine Translationsbewegung in Richtung eines Pfeils 36 aus. Diese Translationsbewegung verläuft parallel und entlang der Längsachse 38 der Spritze 10. Das Einsetzen der Kolbenstange 14 in den Spritzenzylinder 18 ist insoweit den einschlägigen Fachleuten bekannt.

Der Rückanschlag 16 besitzt in dem hier dargestellten Ausführungsbeispiel eine ellipsenförmige Platte 40, die sich quer zur Längsachse 38 der Spritze 10 und damit auch quer zur Kolbenstange 14 erstreckt. Die Platte 40 dient bei zusammengesetzter Spritze 10 als Fingerauflage, und sie erleichtert die Handhabbarkeit der Spritze 10.

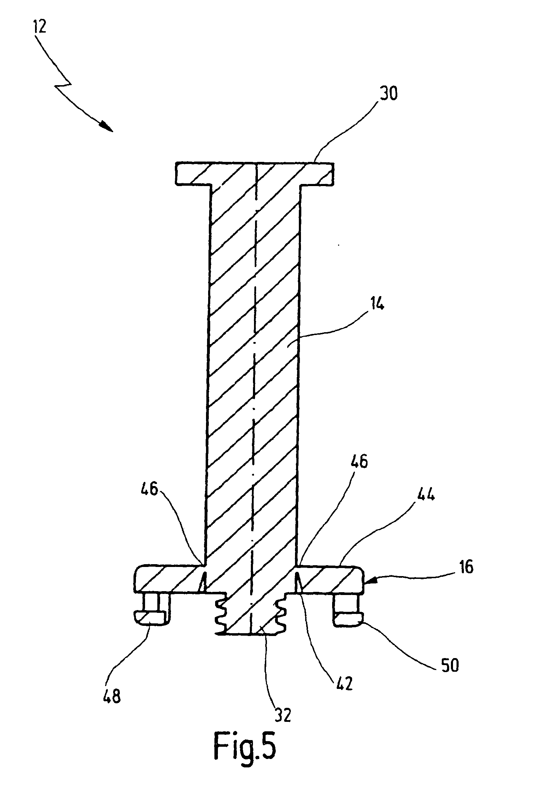

In der Mitte der Platte 40 ist eine ringförmige Nut 42 vorgesehen, die den Gewindezapfen 32 der Kolbenstange 14 konzentrisch umgibt. Wie in Fig. 5 dargestellt, ist die Nut 42 bei dem Montageteil 12, d.h. vor dem endgültigen Zusammenbau der Spritze 10, nicht bis auf den Grund 44 der Platte 40 ausgeführt. Die Nut 42 hat vielmehr die Funktion einer Materialschwächung, die eine Sollbruchstelle zwischen der Kolbenstange 14 und dem Rückanschlag 16 darstellt. Wie nachfolgend näher ausgeführt ist, werden die Kolbenstange 14 und der Rückanschlag 16 bei der Endmontage der Spritze 10 an dieser Sollbruchstelle voneinander getrennt und stellen ab dann zwei separate Bestandteile der Spritze 10 dar.

In einem bevorzugten Ausführungsbeispiel ist die Nut 42 entlang ihres Kreisumfangs teilweise bis auf den Grund 44 der Platte 40 ausgeführt (in der Schnittdarstellung in Fig. 5 nicht zu erkennen), so dass die Kolbenstange 14 und der Rückanschlag 16 entlang der Nut 42 gewissermaßen nur durch kleine Stege 46 miteinander verbunden sind. Durch diese Ausgestaltung lässt sich eine besonders einfache und wirkungsvolle Trennung der Kolbenstange 14 vom Rückanschlag 16 beim Einschrauben der Kolbenstange 14 in den Kolbenstopfen 24 erreichen.

In dem hier bevorzugten Ausführungsbeispiel sind an der zum distalen Ende 20 des Spritzenzylinders 18 zeigenden Seite der Platte 40 zwei Halterungen 48, 50 angeordnet. Die beiden Halterungen 48, 50 bestehen jeweils aus einem U-förmigen Teil, das mit seinen beiden freien Schenkeln einstückig mit der Platte 40 verbunden ist. Die beiden Halterungen 48, 50 bilden damit jeweils eine Einschuböffnung 52 aus, mit deren Hilfe der Rückanschlag 16 am proximalen Ende 26 des Spritzenzylinders 18 befestigt werden kann. Die beiden Halterungen 48, 50 sind in dem hier bevorzugten Ausführungsbeispiel punktsymmetrisch zu dem freien Ende des Gewindezapfens 32 angeordnet.

Das derart ausgebildete Montageteil 12 ist bevorzugt zum Befestigen an Spritzenzylindern 18 mit sogenanntem DIN-Flansch vorgesehen. Ein solcher DIN-Flansch ist in Fig. 2 mit der Bezugsziffer 54 bezeichnet. Charakteristisch für den DIN-Flansch sind vier Ecken, von denen zwei in Fig. 2 mit den Bezugsziffern 56 und 58 bezeichnet sind. Zwei weitere Ecken, die symmetrisch zu den Ecken 56, 58 liegen, greifen in der Darstellung in Fig. 2 in die Einschuböffnungen 52 der beiden Halterungen 48, 50 ein und fixieren den Rückanschlag 16 damit am Spritzenzylinder 18. Da der DIN-Flansch 54 aufgrund der Ecken 56, 58 nicht rotationssymmetrisch ist, wird mit dieser Ausgestaltung eine bajonettartige Verriegelung zwischen dem Rückanschlag 16 und dem Spritzenzylinder 18 realisiert. Diese Art der Verriegelung ist hier insgesamt mit der Bezugsziffer 60 bezeichnet. Zum Verriegeln bzw. Befestigen wird die Platte 40 des Rückanschlags 16 derartige auf den DIN-Flansch 54 aufgesetzt, dass die geradlinigen Seiten 62 des DIN-Flansches 54 parallel zu den beiden Halterungen 48, 50 zu liegen kommen. Durch die Drehbewegung 34, die gleichzeitig zum Einschrauben der Kolbenstange 14 in den Kolbenstopfen 24 dient, drehen sich die in Fig. 2 nicht erkennbaren Ecken des DIN-Flansches 54 in die Einschuböffnungen 52 und verriegeln die Platte 40 am DIN-Flansch 54. Da dieser Verriegelungsvorgang bereits nach einem geringen Drehwinkel abgeschlossen ist, die Kolbenstange 14 zum Einschrauben in den Kolbenstopfen 24 jedoch weiter in Richtung des Pfeils 34 gedreht wird, reißen die Platte 40 und damit der Rückanschlag 16 an den Sollbruchstellen 46 von der Kolbenstange 14 ab. Die Befestigung des Rückanschlags 16 erfolgt damit im selben Arbeitsschritt und mit Hilfe derselben Bewegung, mit der auch die Kolbenstange 14 an dem Spritzenzylinder 18 bzw. dem Kolbenstopfen 24 montiert wird.

Die Darstellung in Fig. 4 zeigt einen Zustand, nachdem der Rückanschlag 16 am Flansch 54 des Spritzenzylinders 18 befestigt und die Kolbenstange 14 von der Platte 40 des Rückanschlags 16 abgerissen wurde. Verbleibende Abrißstellen sind in Fig. 4 mit der Bezugsziffer 64 bezeichnet.

Bei der nachfolgenden Beschreibung eines weiteren bevorzugten Ausführungsbeispiels bezeichnen gleiche Bezugszeichen dieselben Elemente wie zuvor.

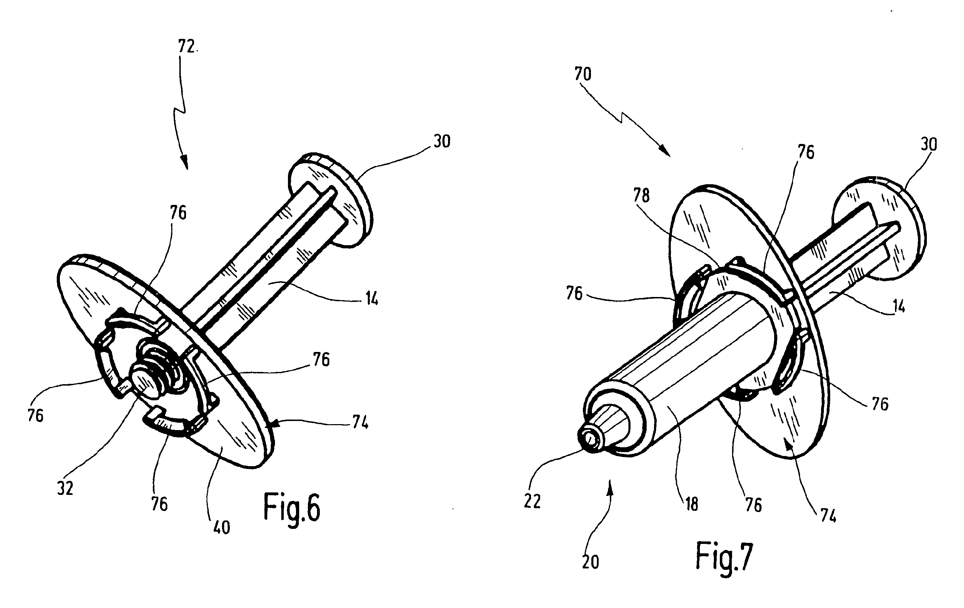

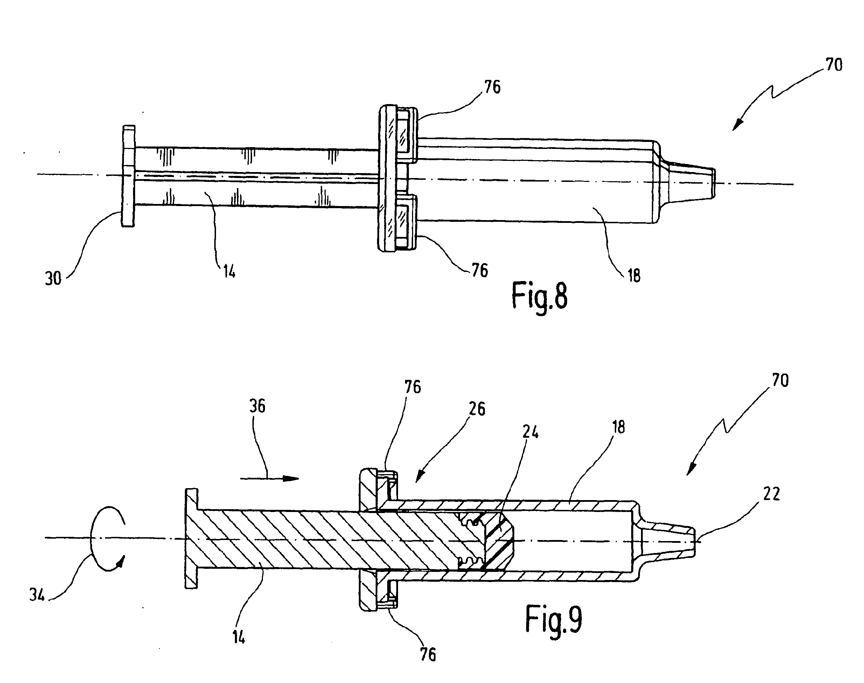

In den Figuren 6 bis 9 ist ein zweites Ausführungsbeispiel der neuen Spritze in seiner Gesamtheit mit der Bezugsziffer 70 bezeichnet. Die Spritze 70 wird unter Verwendung eines Montageteils 72 hergestellt, das sich in der Art seiner Befestigung am Spritzenzylinder 18 von dem Montageteil 12 aus Fig. 1 unterscheidet. Konkret besitzt der hier mit Bezugsziffer 74 bezeichnete Rückanschlag an seiner zum distalen Ende 22 des Spritzenzylinders 18 weisenden Unterseite vier Halterungen 76, die in der Art eines Segmentringes um den Gewindezapfen 32 der Kolbenstange 14 herum angeordnet sind. Die Halterungen 76 bilden Schnapp- bzw. Rastmittel, die bei einem Aufschieben des Rückanschlags 74 auf den Spritzenzylinder 18 in Richtung des Pfeils 36 über den Flansch 78 des Spritzenzylinders 18 greifen. Der Flansch 78 kann bei diesem Ausführungsbeispiel ein DIN-Flansch sein wie in dem vorhergehenden Ausführungsbeispiel, aber auch ein Rundflansch oder eine andere Flanschform. Um das Aufschnappen zu ermöglichen, sind die Halterungen 76, im vorliegenden Ausführungsbeispiel an der Zahl, filigraner und damit flexibler ausgeführt als die Halterungen 48, 50 in dem Ausführungsbeispiel gemäß Fig. 1. Im Übrigen entspricht der Rückanschlag 74 jedoch dem bereits zuvor beschriebenen Rückanschlag 16.

Der Spritzenzylinder 18, Rückanschlag 16 bzw. 74 und Kolbenstange 14 sind in diesen Ausführungsbeispielen aus einem polymeren Werkstoff hergestellt. Dies ermöglicht es, den Rückanschlag 16, 74 und die Kolbenstange 14 in einem einstufigen Spritzgießvorgang herzustellen. Alternativ hierzu ist es auch möglich, den Rückanschlag 16, 74 in einem zweistufigen Spritzgießvorgang an die Kolbenstange 14 anzuformen, so dass unterschiedliche Materialien verwendet werden können. Generell können jedoch auch andere Materialien als polymere Werkstoffe Anwendung finden, beispielsweise könnte der Spritzenzylinder 18 aus Glas hergestellt sein.

In den hier dargestellten, bevorzugten Ausführungsbeispielen sind die Kolbenstange 14 und der Rückanschlag 16, 74 materialschlüssig zu dem kombinierten Montageteil 12, 72 verbunden. Es versteht sich, dass in weiteren Abwandlungen grundsätzlich auch eine andere Verbindung von Rückanschlag und Kolbenstange möglich ist, beispielsweise eine kraftschlüssige und/oder formschlüssige Verbindung. Beispielsweise kann der Rückanschlag 16, 74 eine Bohrung aufweisen, durch die der Gewindezapfen 32 der Kolbenstange 14 hindurchtritt, wobei der Innendurchmesser der Bohrung so gewählt ist, dass die Kolbenstange selbst erst bei Aufbringen einer vorbestimmten Kraft durch die Bohrung hindurch gleiten kann. Des Weiteren kann die zum Einschrauben der Kolbenstange 14 vorgesehene Drehbewegung 34 ausgenutzt werden, um den Rückanschlag 16, 74 aus einer formschlüssigen Verbindung mit der Kolbenstange 14 zu lösen.

In all den genannten Fällen verhindert der Rückanschlag 16, 74 nicht unbedingt, dass die Kolbenstange 14 wieder aus dem Spritzenzylinder 18 herausgezogen werden kann. Durch geeignete Abstimmung der Geometrien, was dem einschlägigen Fachmann ohne Weiteres geläufig ist, kann der Rückanschlag 16, 74 jedoch so ausgebildet werden, dass ein Herausziehen des Kolbenstopfens 24 in Richtung des proximalen Endes 26 des Spritzenzylinders 18 unmöglich ist. Im einfachsten Fall muss lediglich der Innendurchmesser der im Rückanschlag 16, 74 vorgesehenen Durchtrittsöffnung für die Kolbenstange 14 kleiner sein als der Außendurchmesser des Kolbenstopfens 24.

## Patentansprüche

1. Verfahren zum Herstellen einer Spritze (10; 70), insbesondere für medizinische Anwendungen, mit den Schritten:
a) Bereitstellen eines Spritzenzylinders (18) mit einem proximalen (26) und einem distalen (20) Ende,
b) Einsetzen eines Kolbenstopfens (24) in den Spritzenzylinder (18), und
c) Anordnen eines Rückanschlags (16; 74) am proximalen Ende (26) des Spritzenzylinders (18),
wobei der Rückanschlag (16; 74) mit einer Kolbenstange (14) zu einem kombinierten Montageteil (12; 72) verbunden wird und im Schritt c) von der Kolbenstange (14) abgetrennt und am Spritzenzylinder (18) befestigt wird,
**dadurch gekennzeichnet, dass** die Kolbenstange (14) im Schritt c) mit einer Drehbewegung (34) in den Kolbenstopfen (24) eingeschraubt wird, und dass der Rückanschlag (16) mit der Drehbewegung (34) von der Kolbenstange (14) getrennt wird.

2. Verfahren nach Anspruch 1, **dadurch gekennzeichnet, dass** der Rückanschlag (16; 74) im Schritt c) im Wesentlichen parallel zu einer Längsachse (38) des Spritzenzylinders (18) an diesen herangeführt wird.

3. Verfahren nach Anspruch 1 oder 2, **dadurch gekennzeichnet, dass** der Rückanschlag (16; 74) mit der Kolbenstange (14) über Sollbruchstellen (46) derartig verbunden ist, dass der Rückanschlag (16; 74) beim Befestigen der Kolbenstange (14) an dem Kolbenstopfen (24) von der Kolbenstange (14) abreißt.

4. Verfahren nach Anspruch 3, **dadurch gekennzeichnet, dass** der Rückanschlag (16; 74) in einem zumindest zweistufigen Spritzgießvorgang an die Kolbenstange (14) angeformt wird.

5. Verfahren nach einem der Ansprüche 1 bis 4, **dadurch gekennzeichnet, dass** der Rückanschlag (16) mit einer bajonettartigen Verriegelung (60) an dem Spritzenzylinder (18) befestigt wird, wobei die bajonettartigen Verriegelung (60) vorzugsweise zumindest eine Halterung (48, 50) aufweist, in die ein vorspringendes Teil (56, 58) eines an dem Spritzenzylinder (18) angeordneten Flansches (54) eingreift.

6. Verfahren nach einem der Ansprüche 1 bis 5, **dadurch gekennzeichnet, dass** die Kolbenstange (14) beim Befestigen an dem Kolbenstopfen (24) ferner eine Translationsbewegung (36) ausführt, wobei der Rückanschlag (16; 74) mit der Translationsbewegung (36) von der Kolbenstange (14) getrennt wird.

7. Verfahren nach einem der Ansprüche 1 bis 6, **dadurch gekennzeichnet, dass** der Rückanschlag (74) mit Schnappelementen (76), die einen am Spritzenzylinder (18) angeordneten Flansch (78) umgreifen, an dem Spritzenzylinder (18) befestigt wird.

8. Spritze, insbesondere für medizinische Anwendungen, mit einem Spritzenzylinder (18), einem Kolbenstopfen (24), einer daran befestigten Kolbenstange (14) und mit einem Rückanschlag (16; 74) für den Kolbenstopfen (24), wobei der Rückanschlag (16; 74) am proximalen Ende (26) des Spritzenzylinders (18) angeordnet ist, und wobei der Rückanschlag (16; 74) und die Kolbenstange (14) als kombinierbares Montageteil (12; 72) ausgebildet sind, das getrennt an dem Spritzenzylinder (18) angeordnet ist, **dadurch gekennzeichnet, dass** die Kolbenstange (14) mit einer Drehbewegung (34) in den Kolbenstopfen (24) eingeschraubt ist und dass das kombinierte Montageteil (12; 72) so ausgebildet ist, dass der Rückanschlag (16) mit der Drehbewegung (34) von der Kolbenstange (14) abgetrennt ist.

9. Spritze nach Anspruch 8, **dadurch gekennzeichnet, dass** der Rückanschlag (16; 74) und die Kolbenstange (14) jeweils Abrissstellen (64) aufweisen, die zusammen zumindest eine Sollbruchstelle (46) festlegen.

10. Montageteil zum Herstellen einer Spritze, insbesondere für medizinische Anwendungen, mit einer Kolbenstange (14) für die Spritze und einem Rückanschlag (16; 74) zum Befestigen an einem Spritzenzylinder (18), wobei die Kolbenstange (14) und der Rückanschlag (16; 74) zu einem kombinierten Montageteil (12; 72) verbunden sind und zumindest eine vorbereitete Trennstelle (46) aufweisen, **dadurch gekennzeichnet, dass** die Kolbenstange (14) einen Gewindezapfen (32) aufweist, der zum Einschrauben in einen Kolbenstopfen (24) ausgebildet ist, und dass die Trennstelle (46) so ausgebildet ist, dass der Rückanschlag (16) mit einer Drehbewegung (34) von der Kolbenstange (14) abgetrennt werden kann.

11. Montageteil nach Anspruch 10, **dadurch gekennzeichnet, dass** der Rückanschlag (16; 74) und die Kolbenstange (14) konzentrisch zueinander zu dem kombinierten Montageteil (12; 72) verbunden sind.

12. Montageteil nach Anspruch 10 oder 11, **dadurch gekennzeichnet, dass** der Rückanschlag (16; 74) und die Kolbenstange (14) jeweils Abrissstellen (64) aufweisen, die zusammen zumindest eine Sollbruchstelle (46) festlegen.

## Claims

1. A method for manufacturing a syringe (10; 70), in particular for medical applications, comprising the steps of:
a) providing a syringe barrel (18) having a proximal end (26) and a distal end (20),
b) inserting a plunger stopper (24) into the syringe barrel (18), and
c) arranging a backstop (16; 74) at the proximal end (26) of the syringe barrel (18),
wherein the backstop (16; 74) is coupled with a plunger rod (14) to form a combined assembly component (12; 72) and, in step c), is separated from the plunger rod (14) and attached to the syringe barrel (18),
**characterized in that** the plunger rod (14), in step c), is screwed into the plunger stopper (24) with a rotational movement (34), and the backstop (16) is separated from the plunger rod (14) by the rotational movement (34).

2. The method of claim 1, **characterized in that**, in step c), the backstop (16; 74) is guided onto the syringe barrel (18) substantially parallel to a longitudinal axis (38) thereof.

3. The method of claim 1 or 2, **characterized in that** the backstop (16; 74) is coupled with the plunger rod (14) via predetermined break points (46) in such a way that the backstop (16; 74) tears off from the plunger rod (14) when said plunger rod (14) is mounted on the plunger stopper (24).

4. The method of claim 3, **characterized in that** the backstop (16; 74) is formed integrally on the plunger rod (14) in an at least two-step injection-molding process.

5. The method of any of claims 1 to 4, **characterized in that** the backstop (16) is arranged on the syringe barrel (18) with a bayonet-like locking mechanism (60), said bayonet-like locking mechanism (60) preferably having at least one holder (48, 50) into which there engages a projecting part (56, 58) of a flange (54) arranged on the syringe barrel (18).

6. The method of any of claims 1 to 5, **characterized in that** the plunger rod (14) further executes a translational movement (36) when being mounted on the plunger stopper (24), the backstop (16; 74) being separated from the plunger rod (14) with the translational movement (36).

7. The method of any of claims 1 to 6, **characterized in that** the backstop (74) is arranged on the syringe barrel (18) with snap-fit elements (76) which engage around a flange (78) arranged on the syringe barrel (18).

8. A syringe, in particular for medical applications, having a syringe barrel (18), a plunger stopper (24), a plunger rod (14) mounted thereon, and a backstop (16; 74) for the plunger stopper (24), the backstop (16; 74) being arranged at the proximal end (26) of the syringe barrel (18), and wherein the backstop (16; 74) and the plunger rod (14) are designed as a combinable assembly component (12; 72) arranged separately on the syringe barrel (18), **characterized in that** the plunger rod (14) is screwed into the plunger stopper (24) with a rotational movement (34), and the combined assembly component (12; 72) is designed in such a way that the backstop (16) is separated from the plunger rod (14) by the rotational movement (34).

9. The syringe of claim 8, **characterized in that** the backstop (16; 74) and the plunger rod (14) each have tear-off points (64) which together define at least one predetermined break point (46).

10. An assembly component for manufacturing a syringe, in particular for medical applications, comprising a plunger rod (14) for the syringe and a backstop (16; 74) for arranging on a syringe barrel (18), wherein the plunger rod (14) and the backstop (16; 74) are coupled to form a combined assembly component (12; 72) and have at least one prepared separation point (46), **characterized in that** the plunger rod (14) comprises a threaded stub (32) which is designed for being screwed into a plunger stopper (24), and the separation point (46) is designed in a way such that the backstop (16) is separated from the plunger rod by a rotational movement (34).

11. The assembly component of claim 10, **characterized in that** the backstop (16; 74) and the plunger rod (14) are coupled concentrically with respect to one another to form the combined assembly component (12; 72).

12. The assembly component of claim 10 or 11, **characterized in that** the backstop (16; 74) and the plunger rod (14) each have tear-off points (64) which together define at least one predetermined break point (46).

## Revendications

1. Procédé de fabrication d'une seringue (10 ; 70), notamment pour des applications médicales, avec les étapes :
a) mise à disposition d'un cylindre de seringue (18) avec une extrémité proximale (26) et une extrémité distale (20),
b) insertion d'un bouchon de piston (24) dans le cylindre de seringue (18) et
c) agencement d'une butée de renversement (16 ; 74) sur l'extrémité proximale (26) du cylindre de seringue (18),
la butée de renversement (16 ; 74) étant reliée avec la tige de piston (14) en un élément de montage combiné (12 ; 72) et étant séparée dans l'étape c) de la tige de piston (14) et fixée sur le cylindre de seringue (18),
**caractérisé en ce que** dans l'étape c), on visse la tige de piston (14) par un mouvement de rotation (34) dans le bouchon de piston (24) et **en ce que**, par le mouvement de rotation (34), on sépare la butée de renversement (16) de la tige de piston (14).

2. Procédé selon la revendication 1, **caractérisé en ce que** dans l'étape c), on ramène la butée de renversement (16 ; 74) vers le cylindre de seringue (18), sensiblement à la parallèle d'un axe longitudinal (38) de celui-ci.

3. Procédé selon la revendication 1 ou 2, **caractérisé en ce que** la butée de renversement (16 ; 74) est reliée avec la tige de piston (14) via des points destinés à la rupture (46) de sorte que, lors de la fixation de la tige de piston (14) sur le bouchon de piston (24), la butée de renversement (16 ; 74) s'arrache de la tige de piston (14).

4. Procédé selon la revendication 3, **caractérisé en ce qu'**on rapporte la butée de renversement (16 ; 74) dans un procédé de moulage par injection en au moins deux étapes sur la tige de piston (14).

5. Procédé selon l'une quelconque des revendications 1 à 4, **caractérisé en ce qu'**on fixe la butée de renversement (16) sur le cylindre de seringue (18) avec un verrouillage de type baïonnette (60), le verrouillage de type baïonnette (60) comportant de préférence au moins une attache (48, 50), dans laquelle s'engage un élément saillant (56, 58) d'une bride (54) disposée sur le cylindre de seringue (18).

6. Procédé selon l'une quelconque des revendications 1 à 5, **caractérisé en ce que** lors de la fixation sur le bouchon de piston (24), la tige de piston (14) effectue par ailleurs un mouvement de translation (36), la butée de renversement (16 ; 74) se séparant de la tige de piston (14), par le mouvement de translation (36).

7. Procédé selon l'une quelconque des revendications 1 à 6, **caractérisé en ce qu'**on fixe la butée de renversement (74) sur le cylindre de seringue (18) avec des éléments à enclenchement (76), qui entourent une bride (78) disposée sur le cylindre de seringue (18).

8. Seringue, notamment pour des applications médicales, avec un cylindre de seringue (18), un bouchon de piston (24), une tige de piston (14) fixée sur ce dernier et avec une butée de renversement (16 ; 74) pour le bouchon de piston (24), la butée de renversement (16 ; 74) étant disposée sur l'extrémité proximale (26) du cylindre de seringue (18), et la butée de renversement (16 ; 74) et la tige de piston (14) étant conçues sous la forme d'un élément de montage combinable (12 ; 72), qui est disposé séparément sur le cylindre de seringue (18), **caractérisée en ce que** la tige de piston (14) est vissée dans le bouchon de piston (24) par un mouvement de rotation (34) et **en ce que** l'élément de montage combinable (12 ; 72) est conçu de sorte que la butée de renversement (16) se sépare de la tige de piston (14) lors du mouvement de rotation (34).

9. Seringue selon la revendication 8, **caractérisée en ce que** la butée de renversement (16 ; 74) et la tige de piston (14) comportent chacune des éléments de rupture (64), qui ensemble déterminent au moins un point destiné à la rupture (46).

10. Elément de montage pour la fabrication d'une seringue, pour des applications médicales, avec une tige de piston (14) pour la seringue et une butée de renversement (16 ; 74) destinée à être fixée sur un cylindre de seringue (18), la tige de piston (14) et la butée de renversement (16 ; 74) étant reliées en un élément de montage combinable (12 ; 72) et comportant au moins une zone de sectionnement préparée (46), **caractérisé en ce que** la tige de piston (14) comporte un tourillon fileté (32) qui est conçu pour être vissé dans un bouchon de piston (24) et **en ce que** la zone de sectionnement (46) est conçue de sorte que la butée de renversement (16) puisse être séparée de la tige de piston (14) par un mouvement de rotation (34).

11. Elément de montage selon la revendication 10, **caractérisé en ce que** la butée de renversement (16 ; 74) et la tige de piston (14) sont reliées de façon concentrique l'une par rapport à l'autre, pour former l'élément de montage combiné (12 ; 72).

12. Elément de montage selon la revendication 10 ou 11, **caractérisé en ce que** la butée de renversement (16 ; 74) et la tige de piston (14) comportent chacune des zones de sectionnement (64), qui ensemble déterminent au moins un point destiné à la rupture (46).
